# EUROPEAN PATENT APPLICATION

(11) **EP 3 919 067 A1**
(43) Date of publication of application: **08.12.2021**
(21) Application number: 20178105.1
(22) Date of filing: 03.06.2020
(51) Int. Cl.: A61K 38/17, A61P 29/00, A61P 35/00, A61P 37/02

(54) **USE OF SAA, RSAA OR INHIBITORS OF SAA IN THE TREATMENT OF DYSREGULATED INFLAMMATIONS**

(71) Applicant: Lika Antisepsis GbR, 82031 Grünwald (DE)
(72) Inventor: Prof. Dr. Linke, Reinhard, 81475 München (DE)
(74) Representative: Seifert, Ruth

(57) **Abstract**

The present invention describes use of recombinant serum amyloid A proteins (rSAAs) and native serum amyloid A proteins (SAAs) with their natural carrier and an inhibitor of SAA, particularly high density lipoprotein (HDL), in the treatment of a condition or disease that are characterized by dysregulated inflammation. Thereby, in case of dysregulated inflammation with an excessive inflammation, the function of rSAAs and SAAs may be inactivated by binding SAA to HDL at inducing normal temperature or graded hypothermia, leading to an increased amount of SAAs-HDL-complex in said subject *in vivo,* and reversely in case of dysregulated inflammation which has led to low temperatures and hypothermia (suppressed inflammation), rSAA may be administered and/or the SAA level may be increased by separating the SAAs-HDL complex, wherein said step also includes inducing gradual hyperthermia in the subject.

SEQUENCE LISTING

<110> Lika Antisepsis GbR

<120> Use of SAA, rSAA or inhibitors of SAA in dysregulated inflammations

<130> LK20001 EP

<140> EP20178105.1
<141> 2020-06-03

<160> 2

<170> BiSSAP 1.3.6

<210> 1
<211> 122
<212> PRT
<213> Homo sapiens

<220>
<223> SAA1

<400> 1
Met Lys Leu Leu Thr Gly Leu Val Phe Cys Ser Leu Val Leu Gly Val
1               5                   10                  15
Ser Ser Arg Ser Phe Phe Ser Phe Leu Gly Glu Ala Phe Asp Gly Ala
            20                  25                  30
Arg Asp Met Trp Arg Ala Tyr Ser Asp Met Arg Glu Ala Asn Tyr Ile
        35                  40                  45
Gly Ser Asp Lys Tyr Phe His Ala Arg Gly Asn Tyr Asp Ala Ala Lys
    50                  55                  60
Arg Gly Pro Gly Gly Val Trp Ala Ala Glu Ala Ile Ser Asp Ala Arg
65                  70                  75                  80
Glu Asn Ile Gln Arg Phe Phe Gly His Gly Ala Glu Asp Ser Leu Ala
                85                  90                  95
Asp Gln Ala Ala Asn Glu Trp Gly Arg Ser Gly Lys Asp Pro Asn His
            100                 105                 110
Phe Arg Pro Ala Gly Leu Pro Glu Lys Tyr
        115                 120

<210> 2
<211> 122
<212> PRT
<213> Homo sapiens

<220>

<223> SAA2

<400> 2
Met Lys Leu Leu Thr Gly Leu Val Phe Cys Ser Leu Val Leu Ser Val
1               5                   10                  15
Ser Ser Arg Ser Phe Phe Ser Phe Leu Gly Glu Ala Phe Asp Gly Ala
            20                  25                  30
Arg Asp Met Trp Arg Ala Tyr Ser Asp Met Arg Glu Ala Asn Tyr Ile
        35                  40                  45
Gly Ser Asp Lys Tyr Phe His Ala Arg Gly Asn Tyr Asp Ala Ala Lys
    50                  55                  60
Arg Gly Pro Gly Gly Ala Trp Ala Ala Glu Val Ile Ser Asn Ala Arg
65                  70                  75                  80
Glu Asn Ile Gln Arg Leu Thr Gly His Gly Ala Glu Asp Ser Leu Ala
                85                  90                  95
Asp Gln Ala Ala Asn Lys Trp Gly Arg Ser Gly Arg Asp Pro Asn His
            100                 105                 110
Phe Arg Pro Ala Gly Leu Pro Glu Lys Tyr
        115                 120

## Description

The present invention relates to recombinant serum amyloid A proteins (rSAAs) and native serum amyloid A proteins (SAAs) with their natural carrier and inhibitor high density lipoprotein (HDL) (and other inhibitors of SAA) for use in the treatment of a condition or disease that are characterized by dysregulated inflammation in a subject.

A wide range of serious medical conditions, ranging from autoinflammatory diseases, such as inflammatory bowel disease (IBD) or multiple sclerosis (MS), to life-threatening conditions such as sepsis and septic shock, are characterized by dysregulated inflammation.

Sepsis involves an uncontrolled inflammatory response to infection which is a major cause of mortality worldwide. Therapeutic strategies against sepsis, e.g. targeting pro-inflammatory cytokines, have so far shown only limited success. Uncontrolled activation of the immune system can result in systemic inflammatory response syndrome (SIRS) during the course of sepsis, but can also be caused by trauma, burns, pancreatitis, ischemia and hemorrhage. Complications of SIRS and sepsis include organ failure, e.g. acute respiratory distress syndrome (ARDS), multiple organ dysfunction syndrome (MODS), and multiple organ failure syndrome (MOFS), which is associated with poor outcome and high mortality, especially in intensive care patients.

Common treatment of sepsis, SIRS and related conditions involves the use of antibiotics, fluid replacement, surgical drainage of infective fluid collections, and support for organ dysfunction. However, severe complications that are not addressed by these means are often caused by the dysregulated inflammatory response itself, rather than by the infectious agent or trauma. Significant efforts have been undertaken to address these issues, e.g. by targeting key mediators of inflammation, but have yielded rather poor results.

Conditions and diseases that are characterized by chronic inflammation include autoinflammatory and autoimmune disease such as IBD, rheumatoid arthritis, MS and psoriasis. In these conditions, tissue destruction is mediated by chronically active immune mechanisms, which can progress to loss of organ function and severe complications. Moreover, chronic inflammatory organ damage is discussed as a major factor in cancer development and liver cirrhosis.

Acute phase proteins (APPs) are members of a conserved family of proteins that are produced, mainly in the liver, in response to infection and inflammation. They are considered to be a major component of the innate immune response to infection. APPs display a large diversity and can have pro- and anti-inflammatory properties. Accordingly, their role during infections is complex and still remains not entirely understood.

Innate immune responses are critical for host defense against invading pathogens and the subsequent generation of adaptive immune responses. However, pro-inflammatory mediators produced by innate immune cells are also key factors in the pathogenesis of sepsis and related diseases and conditions. Innate immune responses are controlled by complex regulatory pathways involving a multitude of activating and inhibiting mediators. In this context, interleukin 6 (IL-6) is a major pro-inflammatory mediator of sepsis and a strong inducer of APP production in the liver.

Serum amyloid A proteins (SAAs) are important APPs and constitute a highly conserved, multi-gene family of lipoproteins consisting of SAA1, SAA2 and SAA4, the former two of which comprise the acute phase SAAs (A-SAAs). SAA1 and SAA2 are closely related, displaying more than 90% sequence identity to each other. Of note, all SAA molecules in all species tested share invariant clusters of 20 peptides of different length with one of them having the amino acid sequence at positions 33 to 43 which has been shown to mediate major SAA functions.

SAA is associated with a range of chronic and acute inflammatory conditions. In response to acute inflammatory stimuli, e.g. represented by monocyte-derived IL-1, IL-6 and tumor necrosis factor α (TNFα), plasma levels of SAA1 and SAA2 can rapidly increase more than 1000-fold within 6 to 12 hours.

Myeloid derived suppressor cells (MDSCs) represent a heterogeneous, immature population of myeloid cells and precursor cells that can inhibit T cell effector functions. Expansion of MDSCs with T cell suppressive properties has been reported in a mouse model of sepsis. However, these cells' contribution to disease progression and host survival during infections was shown in a murine sepsis model and is otherwise still largely undefined. MDSCs have further been shown to play a protective role in autoinflammatory diseases. Importantly, SAA induces and activates the proliferation of bone marrow cells, including MDSCs. Thus, SAA can induce anti-inflammatory mechanisms via the inductions of MDSCs.

Nevertheless, there is still room for improvement.

The object of the present invention is therefore to provide improved means for the treatment of conditions and diseases that are characterized by dysregulated inflammation.

This object is solved by the subject matters of the independent claims.

In the following use of recombinant serum amyloid A proteins (rSAAs) and native serum amyloid A proteins (SAAs) with their natural carrier and an inhibitor of SAA, particularly high density lipoprotein (HDL), in the treatment of a condition or disease that are characterized by dysregulated inflammation is described. Thereby, the inventor has surprisingly discovered that in case of dysregulated inflammation with an excessive inflammation, the function of rSAAs and SAAs may be inactivated by binding SAA to HDL at inducing normal temperature or graded hypothermia, leading to an increased amount of SAAs-HDL-complex in said subject *in vivo,* and reversely in case of dysregulated inflammation which has led to low temperatures and hypothermia (suppressed inflammation), rSAA may be administered and/or the SAA level may be increased by separating the SAAs-HDL complex, wherein said step also includes inducing gradual hyperthermia in the subject.

Consequently, it has been suggested to use a serum amyloid A protein (SAA), recombinant serum amyloid A protein (rSAA) or an inhibitor of SAA in the treatment of a condition or disease that is characterized by dysregulated inflammation in a subject, wherein the use further comprises the steps of using an inhibitor of SAA in combination with induced hypothermia in the subject, preferably in case of an suppressed inflammation, and using the rSAA and/or SAA in combination with induced hyperthermia in said subject, preferably in case of excessive inflammation.

Thereby, excessive inflammation describes a condition in which a strong acute phase reaction takes place.

Consequently, in case said dysregulated inflammation is suppressed inflammation, said SAA or rSAA is used and said use comprises a step of inducing hyperthermia in said subject, and in case said dysregulated inflammation is excessive inflammation, said inhibitor of SAA is used and said use comprises a step of inducing normal temperature or hypothermia in said subject.

The condition or disease that is characterized by dysregulated inflammation in a subject, to be treated according to the present invention, can fall within two different categories. The first category comprises conditions and diseases wherein said dysregulated inflammation is suppressed inflammation. In this case, the present invention uses SAA or rSAA, wherein rSAA is preferred, and said use comprises a step of inducing hyperthermia in the subject. The second of the above categories comprises conditions and diseases wherein said dysregulated inflammation is excessive inflammation. In this case, the present invention uses inhibitors of SAA, i.e., inhibitors of the subject's endogenous SAA, and said use comprises a step of inducing hypothermia in the subject.

The term "rSAA" as used herein encompasses full-length SAA proteins, fragments thereof having SAA activity, and pharmaceutically acceptable salts thereof. The rSAA used in the present invention is preferably recombinant human serum amyloid A protein (rhSAA). Further, said rSAA is preferably recombinant serum amyloid A1 protein (rSAA1) or recombinant serum amyloid AA2 protein (rSAA2), more preferably recombinant human SAA1 or SAA2 (rhSAA1 or rhSAA2).

In specific embodiments, the rSAA1 comprises or consists of the amino acid sequence according to SEQ ID NO: 1, or an amino acid sequence having at least 90%, at least 90.5%, at least 91%, at least 91.5%, at least 92%, at least 92,5%, at least 93%, at least 93.5%, at least 94%, at least 94.5%, at least 95%, at least 95.5%, at least 96%, at least 96.5%, at least 97%, at least 97.5%, at least 98%, at least 98.5%, at least 99%, or at least 99.5% sequence identity to SEQ NO: 1 and having SAA1 activity. In other specific embodiments, the rSAA2 comprises or consists of the amino acid sequence according to SEQ ID NO: 2, or an amino acid sequence having at least 90%, at least 90.5%, at least 91%, at least 91.5%, at least 92%, at least 92,5%, at least 93%, at least 93.5%, at least 94%, at least 94.5%, at least 95%, at least 95.5%, at least 96%, at least 96.5%, at least 97%, at least 97.5%, at least 98%, at least 98.5%, at least 99%, or at least 99.5% sequence identity to SEQ NO: 2 and having SAA2 activity.

In this context, the term "SAA / SAA1 / SAA2 activity" as used herein relates to said proteins ability to induce and activate the proliferation of bone marrow cells, and, by way of this, induce and activate MDSCs.

The term "inhibitor of SAA" as used herein refers to any inhibitor of endogenous SAA activity, preferably endogenous SAA1 activity and/or SAA2 activity, of the subject. Respective inhibitors include high density lipoprotein (HDL), small molecule, i.e., small organic molecule, inhibitors, bispecific antibodies or more than bispecific peptides of any kind, and inhibitory antibodies, antibody fragments or antibody mimetics directed against SAA. In this context, antibody fragments include Fab fragments, F(ab')₂ fragments, and Fab' fragments. Further, antibody mimetics include single-chain variable fragments (scFv), single-domain antibodies, affibodies, anti-calins, DARPins, monobodies, peptide aptamers, affilins, affimers, and affitins as known in the art.

In specific embodiments, the condition or disease that is characterized by dysregulated inflammation is characterized by excessive inflammation and is selected from the group consisting of sepsis, severe sepsis, septic shock, injury, polytraumatic injury, systemic inflammatory response syndrome (SIRS; e.g. caused by infection, trauma, burns, pancreatitis, ischemia or hemorrhage), multi organ dysfunction syndrome (MODS), multiple organ failure syndrome (MOFS), acute respiratory distress syndrome (ARDS), cancer, liver cirrhosis, Alzheimer's disease, Parkinson's disease, amyotrophic lateral sclerosis (ALS), dementia (such as frontotemporal dementia, vascular dementia, and dementia with Lewy bodies), and autoinflammatory diseases such as inflammatory bowel syndrome (IBD), rheumatoid arthritis, multiple sclerosis (MS), autoimmune hepatitis, acute transplant rejection, graft-versus-host disease, host-versus-graft disease, acute or chronic toxic liver damage, psoriasis, ulcerative colitis, Morbus Crohn, lupus erythematodes, sarcoidosis, severe viral infections (e.g. caused by influenza or corona viruses), and severe chronic bacterial infections.

In other specific embodiments, the condition or disease that is characterized by dysregulated inflammation which is characterized by suppressed inflammation is cancer.

Means for inducing hyperthermia or hypothermia in a subject are not particularly limited and are known in the art. For example, the lowering of the core body temperature in long lasting surgical operations is known and widely practiced.

Preferably, the step of inducing hyperthermia in the subject comprises inducing a body temperature of 37°C or more, 38°C or more, or higher, preferably a body temperature in the range of 37°C or more and 38°C or less, or in the range of 38°C or more and 39°C or less, in the subject. Further, the step of inducing hypothermia in the subject preferably comprises inducing a body temperature of 37°C or less, 36°C or less, or lower, preferably a body temperature in the range of 37°C or less and 36°C or more, or in the range of 36°C or less and 35°C or more, in the subject.

According to the present invention, administration of the SAA, rSAA or inhibitor of SAA and induction of hyperthermia or hypothermia is performed such that the pharmacological action of the SAA, rSAA or inhibitor of SAA and the state of hyperthermia or hypothermia are, at least for a large part, concurrent. Accordingly, the step of inducing hyperthermia or hypothermia can be performed prior to said administration, concurrently with said administration, or after said administration, provided that the above prerequisite of for a large part concurrent action is fulfilled.

Further, formulation, dosage and administration of the SAA, rSAA or inhibitor of SAA according to the present invention, as well as induction of hyperthermia or hypothermia is performed in a manner consistent with good medical practice, as known in the art. Administration can be effected by any suitable means, including parenteral, topical, subcutaneous, intraperitoneal, intrapulmonary, intranasal, and/or intralesional administration. Parenteral infusions include intramuscular, intravenous, intraarterial, intraperitoneal, or subcutaneous administration. Intrathecal administration is also contemplated.

In specific embodiments, the rSAA or inhibitor of SAA used according to the present invention is formulated as a pharmaceutical composition comprising an effective amount of said rSAA or inhibitor of SAA. In further embodiments, said pharmaceutical composition further comprises one or more pharmaceutically acceptable excipients, carriers and diluents.

In a second aspect, the present invention relates to a method for the treatment of a condition or disease that is characterized by dysregulated inflammation in a subject, comprising the steps of administering to the subject an effective amount of a recombinant serum amyloid A protein (rSAA) or an inhibitor of serum amyloid A protein (SAA), wherein
(i) in case said dysregulated inflammation is suppressed inflammation, said rSAA is administered and the method further comprises a step of inducing hyperthermia in said subject, and
(ii) in case said dysregulated inflammation is excessive inflammation, said inhibitor of SAA is administered and the method further comprises a step of inducing normal temperature or hypothermia in said subject.

In this aspect of the present invention, all definitions and limitations defined for the first aspect of the present invention equally apply in an analogous manner.

In a third aspect, the present invention relates to a method for the treatment of a condition or disease that is characterized by dysregulated inflammation in a subject, comprising the steps of
(i) in case said dysregulated inflammation is suppressed inflammation, a step of inducing hyperthermia in said subject, and
(ii) in case said dysregulated inflammation is excessive inflammation, a step of inducing normal temperature or hypothermia in said subject.

In this aspect of the present invention, all definitions and limitations defined for the first aspect of the present invention equally apply in an analogous manner.

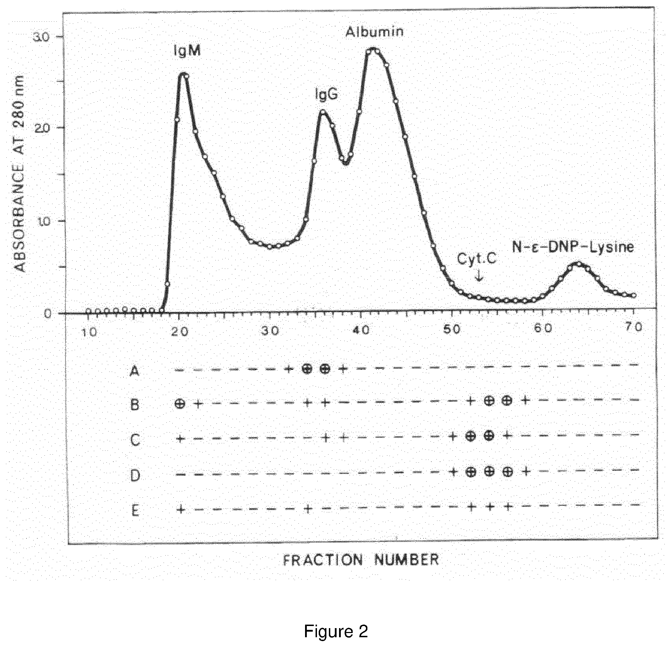

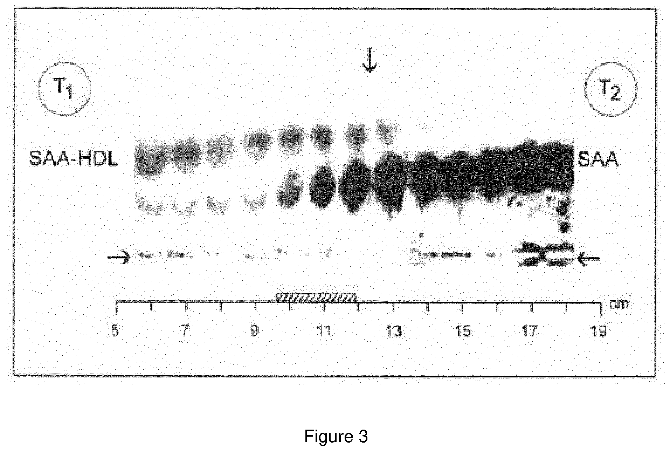

In the present invention, it has been found that inflammatory responses control the action of SAA during dysregulated inflammatory diseases, and that administration of recombinant SAAs or of inhibitors of SAA represents an efficient treatment of such diseases. Further, it has been found that SAA activity is regulated by body temperature-dependent binding of SAAs to high density lipoprotein (HDL), wherein body temperatures of 37°C or lower promote binding of SAA to HDL, and febrile body temperatures of 37°C or above promote SAA release from HDL and activation of SAA (cf. Figs. 2 and 3).

Moreover, it has been found that protective effects of SAAs are mediated at least in parts by promoting the number and activity of MDSC. Thus, SAAs are useful for the treatment of dysregulated inflammatory diseases or conditions.

The present invention relates to the following amino acid sequences:

SEQ ID NO: 1: human SAA1
MKLLTGLVFC SLVLGVSSRS FFSFLGEAFD GARDMWRAYS DMREANYIGS
DKYFHARGNY DAAKRGPGGV WAAEAISDAR ENIQRFFGHG AEDSLADQAA NEW-
GRSGKDP NHFRPAGLPE KY

SEQ ID NO 2: human SAA2
MKLLTGLVFC SLVLSVSSRS FFSFLGEAFD GARDMWRAYS DMREANYIGS
DKYFHARGNY DAAKRGPGGA WAAEVISNAR ENIQRLTGHG AEDSLADQAA
NKWGRSGRDP NHFRPAGLPE KY

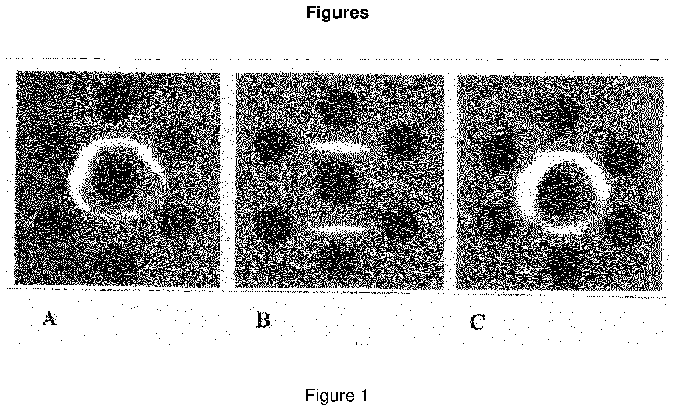

The figures show:
Figure 1:
   Immunochemical comparison of SAA-HDL, SAA and AA.
   Immunodiffusion (ID) at different temperatures. The ID was performed in 1.5% Seakem Agarose in 0.03 M barbital buffer, pH 8.6 with the same reagents in each of the three plates à 6 wells (A-C). Top and bottom well contained AA (0.1 mg/ml), the middle well contained polyclonal rabbit anti-AA antibodies undiluted and the 4 side wells contained elevated SAA-HDL containing APS from 4 patients at 1/10 diluted. Plate A after diffusion over night at room temperature, plate B at 4°C over night and plate C first at 4° over night as plate B at 4°C followed by room temperature for 6 hs similar to plate A.
Figure 2:
   Size separation of SAA-HDL at febrile temperatures.
   SAA-HDL in a patient's acute phase serum with a common cold was separated in A at 37°C, B at 38°, C at 40°c and D at 42°C by gel filtration. All individual fractions (20-70) were examined and semi-quantified by ID using polyclonal rabbit anti-AA antibodies.
Figure 3:
   Electrophoresis of SAA-HDL across a continuous temperature gradient in agarose.
   An APS was applied (see horizontal arrows) in 13 identical samples containing SAA-HDL across a temperature gradient. The small cross-hatched bar on the cm ruler denotes 37°C at the left margin and 42°C at the right one. Electrophoresis was done in 1.5% agarose (Seakem ME) in 25 mm barbital buffer, pH 8.6 followed by a standard Western blot developed with a mixture of the monoclonal anti-AA antibodies (25) mc1+mc4+mc29 at 1+1+1, 1/100. SAA-HDL can be recognized at the left-side site marking the α₁-electrophoretic mobility (upper band). This band fades beginning from 38°C unto 42°C while the SAA freed from HDL (as in Fig. 1) starts to appear at 38°C at the α₂-electrophoretic mobility with the very strong exposure of formerly concealed antigenic AA determinants detected after the strongest exposure appears at febrile temperatures between 38°C and 42°C (see cross-hatched bar). See vertical arrow at 42°C-43°C.

The present invention will be further illustrated by the following example without being limited thereto.

### Examples

### Example 1:

### Identification of the amyloid substance and isolation of its serum precursor

Amyloids of different clinical settings represent characteristic fibrils under electron microscopy. Therefore, for chemical identification of the amyloid, these fibrils had to be extracted in pure form followed by chromatographic isolation of the major amyloid protein for its chemical analysis by amino acid sequence analysis. The method of isolation of the pure amyloid fibrils is known in the art. The first amino acid sequence of an amyloid protein was published in 1970, which was derived from a monoclonal Immunoglobulin κ-light chain and was named ALκ. The first sequence identifying the chemical nature of inflammation-induced amyloid in monkey and human amyloid was published in 1971 which was named amyloid A (AA). The first anti-AA antibodies were prepared in rabbits where a serum protein in patients suffering from inflammations was detected immunochemically. This protein had an α₁-electrophoretic mobility and was in serum approximately of 180 kDa by calibrated gel filtration and thus ready to monitor the isolation of the soluble with anti-AA reactive precursor. This isolation serum protein began in summer 1972 monitored with another rabbit anti-AA antibody. Its chromatographic separation from serum yielded a native 200±20 kDa AA reactive protein which was further chromatographically isolated in 5 M guanidine-HCI. The AA reactive protein had an α₂-electrophoretic mobility and a molecular size of 12.5 kDa. Since this new protein had the same N-terminal amino acid sequence as AA, it was named serum amyloid A (SAA). Since SAA was larger than AA, a limited proteolytic cleavage had to be presumed in order for the former to generate AA. During the isolation of SAA and its purification to one size by gel filtration, by isoelectric focusing, however, eight SAA bands of different isoelectric point named A-H were identified with anti-AA antibodies (with AAE as the major SAA species for the planned radioimmunoassay), thus indicating the first signs of a polymorphism of SAA. In addition, in plasma SAA is bound to HDL.

### Example 2:

### SAA-HDL and febrile temperatures

### Temperature-induced structural changes of SAA in serum

When examining a patient's acute phase serum (APS) with elevated SAA in immunodiffusion (ID) at different temperatures and different times using a polyclonal AA antiserum in comparison with isolated control AA, this resulted in the three different precipitation patterns presented in Fig. 1. In Fig. 1A, one recognizes a line of identity of AA with all four patients' sera as if the SAA (probably SAA1 and SAA2) reaction were done with pure SAA. At 4°C in Fig. 1B, however, there is no reaction with SAA-HDL in serum. This is due to the hiding of the AA-reactive parts of SAA through HDL. However, when the temperature was switched to room temperature after the reaction in Fig. 1B at 4°C, the SAA containing serum resulted in a strong line after releasing the SAA from HDL in Fig. 1C as seen in Fig. 1A. However, different from the pattern in Fig. 1A, the precipitation line of AA-Anti-AA is somewhat independent of the SAA-anti-AA line, thus indicating that the homologous AA-Anti-AA line seems to be more stable than the SAA-Anti-AA line. These results show that SAA-HDL is stable in full APS at 4°C where the AA-reactive sites are covered by HDL. When at room temperature (in ID buffer), where SAA is released from HDL and is now accessible to antibodies for precipitation, it is reactive. Therefore, the separation of SAA from HDL is temperature-dependent. These results became only fully explainable through the next section, where the separation of SAA from HDL became clear. In addition, recombinant SAA2 was prepared and, when added to normal human serum, it was possible to repeat exactly that behavior reported in Fig. 1. This shows that SAA alone can reproduce this phenomenon.

### The molecular size of the SAA and SAA-HDL at different febrile temperatures

Temperature-dependent molecular weight determination of AA-antigenic proteins of acute phase serum (APS) has been performed using an ACA-34 gel filtration column in PBS with the enzyme inhibitor phenylmethylsulfonylfluoride (PMSF) under various temperatures as shown in Fig. 2. The size grading was done by the serum proteins IgM, IgG, albumin and, in addition, by cytochrome C and the salt marker N-ε-DNP-lysine. The proteins were identified by way of the size position in the column by immunodiffusion as SAA-HDL at a size of ca. 180 - 200 kDa or SAA at 12.5 kDa. The different temperatures were kept with a temperature-controlled glass jacket, that is at 37°C in column run A, at 38°C in B, at 40°C in C and at 42°C in D. E was run as D, but without enzyme-protection by PMSF, thus showing some degradation of SAA.

At a normal body temperature of 37°C, AA-containing proteins are at a single position as that of the SAA-HDL stable complex in A (fractions 34-37). However, already at 38°C, the stable complex SAA-HDL begins to dissociate as shown in Fig. 2, run B. AA antigenic proteins appear at three positions, that is first of all at the void volume at fractions 19-20 (which has not been further analyzed, but could be related to aggregated SAA and/or its derivatives), secondly at the position of the stable SAA-HDL complex at fractions 34-36 and thirdly at the position of the HDL-free SAA at 53-56, as determined by the antigenic differentiation as seen in Fig. 1. This size differentiation may also indicate functional heterogeneity, as the different affinities of SAA to HDL. This dissociation begins at 38°C and progresses with diminution of the SAA-HDL complex until run C. SAA-HDL disappeared at a "threshold of life" in run D at 42°C and above where the SAA species was maximized and the broadest was seen at fraction 53-56. This shows a temperature-induced gradual dissociation of SAA from HDL at the different febrile temperatures, which was shown here *in vitro.* This may also occur under systemic and local, acute phase conditions, with the release of different SAA isotypes at different temperatures, for functions to be discovered. Finally, the SAA monomers released at different temperatures differ in size. SAA in B is somewhat smaller than SAA in C. In addition, both appear at 42°C in D together as a broad combination of the two SAAs in B and D. In conclusion, SAA separated from HDL at 38°C in B has a lower affinity to HDL and is smaller, and SAA with a higher affinity for HDL is larger. Different isotypes and sizes of SAA are known. The acute phase SAAs, aSAA1 and aSAA2, are each 12.5 kDa with 104 amino acids and the constitutive SAA (cSAA) which is 14 kDa and has 112 amino acids. Since SAA1 has the lowest affinity for HDL and is the most amyloidogenic SAA, it could have separated from HDL in run at 38°C in B already while SAA4, which is somewhat larger than the aSAAs, could be a component in C. These indications can be solidified using isoelectric focusing or SAA-isotype-specific antibodies.

### Gradual dissociation of SAA-HDL during a continuous temperature gradient

While the experiments above were done stepwise, one by one, a more precise dissociation of the SAA-HDL separation was performed by electrophoresis in 1.5% agarose across a continuous temperature gradient in a single flat gel, as shown in Fig. 3. The two sides between the agarose gel were kept at a constant temperature of 15°C in T1 and of 65°C in T2.

The results in Fig. 3 show two horizontal bands of samples of one patient in the form of dots across the temperature gradient. The SAA-HDL band of α₁-electrophoretic mobility is marginally stained due to the concealing of the AA-antigenic determinants of SAA within the SAA-HDL complex at low temperatures. The hiding of the antigenic determinants disappeared gradually from 38°C (the 5^{th} sample) until it is completely above 42°C with the appearance of large amounts of SAA (intensive staining of the band with α₂-mobility). By gradually increasing the temperature, the SAA release increases gradually while the SAA-HDL fades away up to the extreme exposure at 42°C and beyond, in agreement with the stepwise separation of SAA from HDL shown in Fig. 2. Also important during this gradual temperature-induced separation from HDL seems to be that the morphology of the dots is different. They changed by their shape in the longitudinal direction which is consistent with the fact that SAA is not uniform and consists of a group of homologous, but chemically different SAAs demonstrating different isoelectric points. Another observation concerns the AA-antigenic species below 37°C in the first four samples. These slow, arc-like uniform samples could represent an SAA species which is always active as a monomer regardless of temperature. This species seems to be less acidic. It could be a type of SAA species for the general protection under normal condition and may be therapeutically useful.

In sample 5, this "arc SAA" is overlaid by a more acidic SAA released from the acute phase proteins (APPs) SAA1 or SAA2, thus changing the spots to a more longitudinal pattern. With increasing temperature, the SAA spots become thicker and increase more in the longitudinal direction.

### Activation of the SAA by separation from HDL under febrile temperatures and consequences

Taken together, it is clear that the mechanism of separation of SAA from HDL *in vitro* is also strictly regulated *in vivo* by body temperatures above 37°C. Therefore, this is a key mechanism which can be induced and activated basically by two different manifestations. The most common is the orthologic APR activation of SAA. This occurs with a maximal SAA concentration of up to 1000 times within a day as a systemic "biochemical thunderstorm" with a myriad of activating and inhibiting events simultaneously, which are not understood in detail today. During these events, the cause of the APR will be eradicated and the APR becomes curative.

With this beneficial outcome, the normal immune homeostasis returns in a foreseeable future. However, when the APR cannot overcome its initial cause it will become a pathologic APR with a "persistent biochemical thunderstorm" and lacking a self-driven cure. The consequences can be summarized in an exhaustion of the resources of the organism and decline of the metabolic activity through a multitude of clinically challenging conditions exemplified by severe viral and bacterial chronic inflammations, systemic inflammatory response syndrome (SIRS) or uncontrolled chronic infections, sepsis and septic shock. Moreover, when the infection remains limited, a local APR will take care of it.

The functions of the four human isotypes, SAA1, SAA2, (SAA3 in humans is only transcribed in some cells) and the SAA4 have not been fully analyzed. They have arisen through gene duplications, thus indicating important individual functions either alone or in combination. As described before, the human acute phase A-SAA has two very similar isotypes, A-SAA1 and A-SAA2, in the APR mostly synthesized in the liver and expressed in most body cells (cf. below) and the constitutive C-SAA4 and some allotypes in SAA1 and SAA2.

Another discovery was the discontinuous separation of SAA from HDL described above at different temperatures, meaning that not all SAA molecules are being separated from HDL at a single temperature except for the temperature of 42°C (Fig. 2) and above (Fig. 3). In fact, these figures show that the separation of SAA is spreading out over the whole febrile temperature range starting from 38°C to 42°C and above. In addition, based on these observations in Fig. 3, it is possible that SAA isotypes and allotypes are separated from HDL at different febrile temperatures and thereafter fulfill their different functions locally or systematically as individual SAA species as is also to be derived from Fig. 2. Another indication for the differential release of the SAA species can be detected in Fig. 3 in the different shapes of the protein blots of the SAAs devoid of HDL, thus indicating possible SAAs with distinct isoelectric points (IP). In Fig. 3 there are free dots before 37°C named (for convenience) "arc SAA", the least acidic SAA. The SAA species released from HDL after 37°C ("38 SAA") are probably the more acidic ones. In this sense, the dot changes also occur later on 39°C-, 40°C-released SAA, etc. Analyzing the spots for the identity of the various SAA species could show whether these indications did discover a mechanism by which the different SAA species can be released from HDL and thereby are being activated at specific temperatures alone or with other SAAs for special purposes which need to be analyzed. These points may also be of therapeutic interest. This proposed temperature-selection of SAA isotypes could specify the needed APR function for a specific purpose. The increase of the organism's temperature is being induced by the organism as a response to various stimuli, exemplified by bacterial invasion. It could represent some sort of a "gear shift" for providing a graded response in order to release special SAAs to provide adequate amounts which are necessary "tools" for survival. This could occur in concert with other agents including other APPs and cytokines of the APR network. The possible therapeutic manipulation of the body's temperature ("the gear shift") *in vivo* needs the precise analysis of this phenomenon *in vivo* first.

### The multifunctional SAA family

Various functions of SAA have been reported, including the systemic and local elevation of SAA in inflammations in an APR due to the systemic and local cytokine increase. SAA is involved in very many functions as being an opsonin of gram-negative bacteria, a chemoattractant, an inducer of chemokines and cytokines, a stimulator of angiogenesis, important in cholesterol transport and a modulator in the migration of white blood cells. SAA acts concentration-dependently on polymorphonuclear cells and the degradation of SAA, which can release the AA 1-76 fragment and can thereby induce the fatal AA-amyloidosis in humans and animals. Other fragments of SAA and other APPs may, *in vivo,* influence this still not understood complex network of the SAA family. Here, some of these vital functions of SAA have been identified by blocking these functions by way of monoclonal AA/SAA antibodies.

### HDL binding site

The HDL binding site of SAA was identified as the peptide aa 5-17 with the monoclonal mc1. The presumptive estimate was aa 1-11.

### Human neutrophils

Strong binding of isolated, acute-phase human SAA (and recombinant SAA2, not presented) were shown with human neutrophils assuming the existence of an SAA receptor which may have regulatory functions. The FMLP-induced oxidative burst of normal human neutrophils could be reduced, concentration-dependently, by SAA at concentrations of 0.1 µg/ml and 1.0 µg/ml. This inhibitory reduction of SAA could be blocked by the monoclonal antibody mc29, which binds to the synthetic peptide aa 28-40 of SAA, thus proving that this blocked area is responsible for this inhibitory effect of SAA. This was the first time that a function of SAA was blocked by a monoclonal AA antibody. The monoclonal antibody mc29 used probably also blocks the laminin-like domain (aa 29-33) and may also be participating partially with the RGD-like domain (aa 39-41). In addition, human neutrophils were exposed to full human APS at different temperatures. At 41°C, the inhibition of the oxidative burst was much stronger than at 37°C, indicating the role of SAA freed from HDL and in its active state (Figs. 2 and 3). However, when the acute phase serum was preheated to 41°C for 15 min and assayed at 37°C, the SAA-containing serum did not return to the 37°C value, but stayed with the increased 41°C inhibiting effect at 37°C. This indicated an irreversible structural change of SAA (or its fragments) during high fever, which is blocking SAA's return to the reversible binding to HDL. (This febrile temperature-that induced the aggregation of AA-antigenic proteins has also been noticed *in vitro* and documented in Fig. 2 at 38°C and 40°C). The possibly unfavorable consequences of these aggregates in humans or animals are unknown today.

### Anti-inflammatory potential of SAA on neutrophils

The anti-inflammatory potential of SAA on neutrophils has been confirmed for SAA at reported serum concentrations. Oxidative burst, migration and the neutrophil myeloperoxidase release were also inhibited. SAA peptides (aa 1-14, 15-101 and 83-104) also contributed to this inhibitory effect. However, at higher concentrations of more than 50 µg/ml, SAA was stimulating. In addition, O₂-release was inhibited up to 0.1 µg/ml, but the O₂-release was increased above that. Thus, SAA plays a dual role, it down-regulates inflammatory processes in lower concentration but, during the full APR, the action of SAA can be promoting.

### Presence of SAA in human cancer and other cells

Intracellular SAA of colon tissue with cancer of progressing stages of anaplasia was examined on formalin-fixed paraffin sections from 26 patients with colon cancer (after SAA plasma levels were shown by others to be elevated in carcinomas, assuming that the elevated SAA is of hepatic origin). SAA was detected immunohistochemically by using the monoclonal antibodies mc1 and mc29. On normal cells, no reaction or only traces were detected. However, stronger reactions were found in carcinoma cells. The staining intensity increased gradually from dysplasia to the stage of malignant neoplasia. The metastases also showed the presence of SAA, but weaker. In addition, cells, other than colon cells in these sections, also showed the presence of SAA as lymphoid cells of the intestinal wall, inflammatory cells, ganglion cells and endothelial cells. The presence of SAA has been confirmed by *in situ* hybridization and reverse transcriptase polymerase chain reaction (RT-PCR). The genes of SAA1 and SAA4 in the colon carcinomas were activated. Although the role of SAA in colon carcinoma is unknown, the close association of the increasing grade of malignancy with the increased SAA synthesis may indicate a role of SAA in tumorigenesis. SAA can serve as an adhesive ligand for tumor-cell homing, it induces inflammation, which may be neoplastic. It also induces migration and can be involved in metastasis, or it can be inhibitory to attachment.

### Example 3:

### Therapeutic change of body temperature

It is important to define the various febrile temperatures by which the individual SAAs separate from HDL (proven *in vitro,* Figs. 2 and 3) and getting activated to execute their individual function which needs to be defined. A novel idea, underlying the present invention, is the therapeutic application of normothermic to hypothermic temperatures for inactivation of SAA through binding to HDL which can be called "hypothermic deactivation of SAA". This option could be considered (after complying with the strict rules for a novel therapy) in severe inflammatory states when SAA is detrimental, exemplified by chronic febrile autoimmune diseases such as lupus erythematodes and devastating chronic inflammations such as sepsis, septic shock, genetic hyperthermia syndromes and similar diseases summarized in chronic SIRS (systemic inflammatory response syndrome). Inversely, in a low temperature-dependent blockage of SAA by HDL conformational change of SAA at 38°C and above causing SAA release from HDL could induce a common "hyperthermia activation of SAA", which could be beneficial for individual patients with diseases having clinical syndromes with body temperatures of 37°C and below.

## Claims

1. Serum amyloid A protein (SAA), recombinant serum amyloid A protein (rSAA) or an inhibitor of SAA for use in the treatment of a condition or disease that is **characterized by** dysregulated inflammation in a subject, wherein
(i) in case said dysregulated inflammation is suppressed inflammation, said SAA or rSAA, preferably said rSAA, is used and said use comprises a step of inducing hyperthermia in said subject, and
(ii) in case said dysregulated inflammation is excessive inflammation, said inhibitor of SAA is used and said use comprises a step of inducing normal temperature or hypothermia in said subject.

2. SAA, rSAA or inhibitor of SAA for use according to claim 1, wherein said rSAA is recombinant human serum amyloid A protein (rhSAA).

3. SAA, rSAA or inhibitor of SAA for use according to claim 1 or claim 2, wherein said rSAA is recombinant serum amyloid A1 protein (rSAA1) or recombinant serum amyloid A2 protein (rSAA2).

4. SAA, rSAA or inhibitor of SAA for use according to claim 3, wherein said rSAA1 comprises
(i) the amino acid sequence according to SEQ ID NO: 1, or
(ii) an amino acid sequence having at least 90% sequence identity to SEQ NO: 1 and having SAA1 activity; and
said rSAA2 comprises
(i) the amino acid sequence according to SEQ ID NO: 2, or
(ii) an amino acid sequence having at least 90% sequence identity to SEQ NO: 2 and having SAA2 activity.

5. SAA, rSAA or inhibitor of SAA for use according to claim 4, wherein said rSAA1 consists of
(i) the amino acid sequence according to SEQ ID NO: 1, or
(ii) an amino acid sequence having at least 90% sequence identity to SEQ NO: 1 and having SAA1 activity; and
said rSAA2 consists of
(i) the amino acid sequence according to SEQ ID NO: 2, or
(ii) an amino acid sequence having at least 90% sequence identity to SEQ NO: 2 and having SAA2 activity.

6. SAA, rSAA or inhibitor of SAA for use according to any one of claims 1 to 5, wherein said inhibitor of SAA is high density lipoprotein (HDL) and/or selected from the group consisting of small molecule inhibitors, bispecific antibodies, bispecific peptides, and inhibitory antibodies, antibody fragments or antibody mimetics directed against SAA.

7. SAA, rSAA or inhibitor of SAA for use according to any one of claims 1 to 6, wherein said inhibitor of SAA is an inhibitor of serum amyloid A1 protein (SAA1) and/or serum amyloid A2 protein (SAA2).

8. SAA, rSAA or inhibitor of SAA for use according to any one of claims 1 to 7, wherein
(i) the condition or disease that is **characterized by** dysregulated inflammation is **characterized by** excessive inflammation and is selected from the group consisting of sepsis, severe sepsis, septic shock, injury, polytraumatic injury, systemic inflammatory response syndrome (SIRS; e.g. caused by infection, trauma, burns, pancreatitis, ischemia or hemorrhage), multi organ dysfunction syndrome (MODS), multiple organ failure syndrome (MOFS), acute respiratory distress syndrome (ARDS), cancer, liver cirrhosis, Alzheimer's disease, Parkinson's disease, amyotrophic lateral sclerosis (ALS), dementia (such as frontotemporal dementia, vascular dementia, and dementia with Lewy bodies), and autoinflammatory diseases such as inflammatory bowel syndrome (IBD), rheumatoid arthritis, multiple sclerosis (MS), autoimmune hepatitis, acute transplant rejection, graft-versus-host disease, host-versus-graft disease, acute or chronic toxic liver damage, psoriasis, ulcerative colitis, Morbus Crohn, lupus erythematodes, sarcoidosis, severe viral infections (e.g. caused by influenza or corona viruses), and severe chronic bacterial infections; or
(ii) the condition or disease that is **characterized by** dysregulated inflammation is **characterized by** suppressed inflammation and is cancer.

9. SAA, rSAA or inhibitor of SAA for use according to any one of claims 1 to 8, wherein the step of inducing hyperthermia in the subject comprises inducing a body temperature of 38°C or more in the subject, and
wherein the step of inducing hypothermia in the subject comprises inducing a body temperature of 37°C or less in the subject.

10. SAA, rSAA or inhibitor of SAA for use according to any one of claims 1 to 9, wherein the rSAA or inhibitor of SAA is formulated as a pharmaceutical composition comprising an effective amount of said rSAA or inhibitor of SAA.

11. SAA, rSAA or inhibitor of SAA for use according to claim 10, wherein the pharmaceutical composition further comprises one or more pharmaceutically acceptable excipients, carriers and diluents.

12. Method for the treatment of a condition or disease that is **characterized by** dysregulated inflammation in a subject, comprising the steps of administering to the subject an effective amount of a recombinant serum amyloid A protein (rSAA) or an inhibitor of serum amyloid A protein (SAA), wherein
(i) in case said dysregulated inflammation is suppressed inflammation, said rSAA is administered and the method further comprises a step of inducing hyperthermia in said subject, and
(ii) in case said dysregulated inflammation is excessive inflammation, said inhibitor of SAA is administered and the method further comprises a step of inducing normal temperature or hypothermia in said subject.

13. Method according to claim 12, wherein the step of inducing hyperthermia or hypothermia is performed prior to said administration, with said administration, or after said administration.
